# EUROPEAN PATENT SPECIFICATION

(11) **EP 1 618 926 B1**
(45) Date of publication and mention of the grant of the patent: **03.06.2009**
(21) Application number: 03816647.6
(22) Date of filing: 14.04.2003
(51) Int. Cl.: A61K 8/81, A61Q 5/12

(54) **HAIR TREATMENT COMPRISING ACRYLIC/SILICONE COPOLYMER AND COSMETIC HAIR PREPARATION CONTAINING THE TREATMENT**
HAARBEHANDLUNG MIT ACRYL-/SILIKON-COPOLYMER UND DIESE BEHANDLUNG ENTHALTENDES HAARPFLEGEPRÄPARAT
TRAITEMENT CAPILLAIRE COMPRENANT UN COPOLYMERE ACRYLIQUE/SILICONE ET PREPARATION CAPILLAIRE COSMETIQUE CONTENANT CE TRAITEMENT

(43) Date of publication of application: 25.01.2006
(73) Proprietor: Shin-Etsu Chemical Co., Ltd., Tokyo 100-0004 (JP)
(72) Inventor: ONO Ichiro c/o Silicone Denshi Zairyo Gijutsu, Matsuida-machi Annaka-shi Gunma-ken (JP); TACHIBANA, Kiyomi, c/o Shin-Etsu Chemical Co. Ltd., Tokyo 100-0004 (JP)
(74) Representative: Winckels, Johannes Hubertus F.
(86) International application number: PCT/JP2003/004706
(87) International publication number: WO 2004/091563

(56) References cited:
- WO-A1-98/54255
- WO-A1-02/100356
- JP-A- 4 321 618
- DATABASE WPI Section Ch, Week 200582 Derwent Publications Ltd., London, GB; Class A26, AN 2002-150729 XP002385655 & JP 2001 226236 A (GYOKU RIKAGAKU KK) 21 August 2001 (2001-08-21)

## Description

### FIELD OF THE TECHNOLOGY

This invention relates to an organopolysiloxane hair treatment agent and a hair cosmetic comprising the treatment agent. The hair treatment agent increases effects of applying hair rinse, hair conditioner or hair treatment product to the hair and improves durability of the effects.

### BACKGROUND OF THE INVENTION

Nowadays people wash hair everyday and casually perm or color. Hair damages physically or chemically caused by the hair wash, perm or color are serious problems. For damaged hair, conditioning is necessary. As a conditioning agent, oils, cationic surfactants, proteins and hydrolysates thereof, moisturizer, higher alcohols, and emulsions are used. However, conditioning effects of the oils, cationic surfactants, proteins and hydrolysates thereof, moisturizer, higher alcohols, and emulsions are lost by a hair wash and not durable.

A hair treatment agent has been strongly desired which resists hair wash to maintain effects of the conditioning. As a method to maintain conditioning effects, Japanese Patent Application Laid-Open No.2001-226236 discloses a method to treat the hair with a methylhydrogenpolysiloxane. The treatment with the polysiloxane is indeed effective but not sufficient. Moreover, unreacted Si-H bonds remain after the treatment, which sometimes cause generation of hydrogen gas. Thus, a hair treatment agent is desired which maintains hair conditioning effect.

### DISCLOSURE OF THE INVENTION

As a result of intensive studies, the present inventors have found that the aforesaid problem of the conventional treatment agents are solved, that is, conditioning effect is improved and retained, by using an acryl/silicone copolymer hair treatment agent and a hair cosmetic comprising the treatment agent.

Thus, the present provides a hair treatment agent comprising an acryl/silicone copolymer (A) having at least one hydrolyzable silyl group per molecule and a hair cosmetic comprising the hair treatment agent. Preferably, the hydrolyzable silyl group is -Si(CH₃)₃₋ₘ(OR)ₘ

The present invention also provides a two-agent hair cosmetic kit composed of a first agent comprising at least one selected from the group consisting of amino-modified silicones, amino acid-modified silicones, and carboxyl-modified silicones, and a second agent comprising the aforesaid hair treatment agent, and a three-agent hair cosmetic kit composed of the aforesaid hair cosmetic kit and an amino-modified silicone.

The aforesaid cosmetic may further comprise at least one selected from an unctuous agent, water, a compound having an alcoholic hydroxyl group, a water-soluble or water-swelling polymer, a surfactant, resins such as a crosslinked organopolysiloxane, and powder and/or coloring agent.

The aforesaid cosmetic is in the form of liquid, milky lotion, cream, solid, paste, gel, multilayer, mousse, spray or stick.

### PREFERRED EMBODIMENTS OF THE INVENTION

A hair treatment agent of the present invention comprises an acryl/silicone copolymer having at least one hydrolyzable silyl group per molecule. The acryl/silicone copolymer may be of any type such as a block copolymer or a graft copolymer as long as it has at least one hydrolyzable silyl group per molecule. Preferably, the acryl/silicone copolymer is an acryl/silicone graft copolymer having an acrylic polymer backbone and an organopolysiloxane polymer branch.

Preferred hydrolyzable silyl group has a -Si-O- moiety, for example, an alkoxysilyl group or acetoxysilyl group. More preferred silyl group is represented by the formula, -Si (CH₃)₃₋ₘ(OR)ₘ, wherein R is an alkyl, alicyclic, alkenyl, aryl, or aralkyl group, preferably an alkyl or alkenyl group having 1 to 4 carbon atoms. Most preferably, R is an ethyl group. In the above formula, m is an integer of from 1 to 3. When m is 2 or 3, a plurality of R's may be different from each other. Example of preferred hydrolyzable silyl group include dimethylethoxysilyl group, methyldiethoxysilyl group and triethoxysilyl group, among which triethoxysilyl group is particularly preferred.

Preferably, the acryl/silicone copolymer has the following repeating units of (1), (2) and (3).

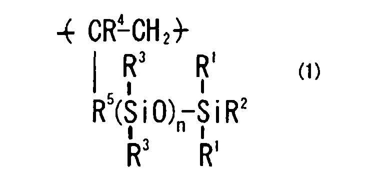

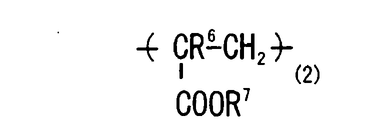

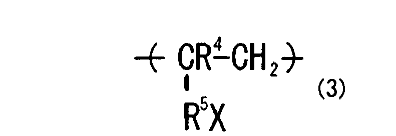

In the repeating units of from (1) to (3), R1, R² and R³ may be the same with or different from each other and are selected from the group consisting of alkyl groups having 1 to 30 carbon atoms, aryl groups, aralkyl groups and fluorinated alkyl groups,
R⁴ and R⁶ may be the same with or different from each other and are selected from the group consisting of a hydrogen atom and a methyl group,
R⁵ may be the same with or different from each other and is an alkyleneoxycarbonyl group having 2 to 11 carbon atoms or a phenylene group,
R¹ is an alkyl group having 1 to 30 carbon atoms,
X is the hydrolyzable silyl group as described above, and
n is an integer of from 3 to 500.

The acryl/silicone graft copolymer can be prepared by reacting an acrylic polymer with an organopolysiloxane. Preferably, for the reason of easiness in production and molecular design, so-called macromonomer method is employed by copolymerizing an organopolysiloxane represented by the following formula (4) having a radically polymerizable group, an acrylic monomer represented by the following formula (5), and a silane compound represented by the following formula (6) having a radically polymerizable group, 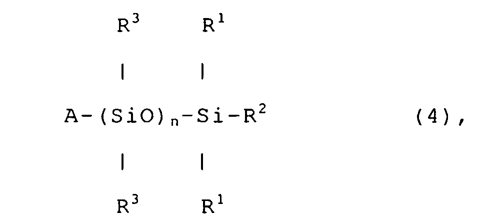 wherein R¹, R² and R³ may be the same with or different from each other and are selected from the group consisting of alkyl groups having 1 to 30 carbon atoms, aryl groups, aralkyl groups and fluorinated alkyl groups, Ais a radicallypolymerizable group represented by the following formula (7),

CH₂=C(R⁴)R⁵- (7),

wherein R⁴ is a hydrogen atom or a methyl group and R⁵ is an alkyleneoxycarbonyl group having 2 to 11 carbon atoms or a phenylene group, and
n is an integer of from 3 to 500;

CH₂=C(R⁶)COOR⁷ (5),

wherein R⁶ is a hydrogen atom or a methyl group and R⁷ is an alkyl group having 1 to 30 carbon atoms;

B-Si(CH₃)₃₋ₘ(OR⁸)ₘ (6),

wherein B is selected from the groups defined for A above, independently of A, R⁸ is an alkyl group having 1 to 4 carbon atoms or an alkenyl group, m is an integer of from 1 to 3, and R^{B}'s may be different from each other when m is 2 or 3.

The organopolysiloxane compound represented by the formula (6) having a radically polymerizable group is called a silicone macromonomer. It has the radically polymerizable group at one end only. From the compound, the repeating unit (1) can be derived. In the formula (4), A represents a monovalent organic group having a radically polymerizable group and is represented by the formula (7) described above. Examples of A include (meth)acryloxymethyl group, (meth)acryloxypropyl group, (meth)acryloxydecyl group, styryl group, and α-methylstyryl group, among which a (meth)acryloxypropyl group is preferred.

In the formulae (1) and (4), examples of R¹ include alkyl groups such as methyl, ethyl, propyl, butyl, pentyl, hexyl, heptyl, octyl, nonyl, decyl and cyclohexyl groups; aryl groups such as phenyl and tolyl groups; aralkyl groups such as benzyl and phenetyl groups ; and fluorinated alkyl groups such as trifluoropropyl and nonafluorobutylethyl groups. Preferably, most of R¹ are methyl groups because of good feel to the touch.

In the formulae (1) and (4), n is an integer of from 3 to 500, preferably from 9 to 200. An acryl/silicone copolymer with n less than the aforesaid lower limit may not provide satisfactory conditioning effect. An organopolysiloxane compound with n above the aforesaid upper limit may not copolymerize with the acrylic monomer or the radically polymerizable silane compound with ease to make it difficult to obtain a desired copolymer.

From the acrylic monomer of the formula (5), the repeating unit (2) can be derived. Examples of the monomer include methyl (meth)acrylate, ethyl (meth)acrylate, isopropyl (meth)acrylate, n-butyl (meth)acrylate, tert-butyl (meth)acrylate, cyclohexyl (meth)acrylate, 2-ethylhexyl (meth)acrylate, tridecyl (meth)acrylate, stearyl (meth)acrylate, isostearyl (meth)acrylate, behenyl (meth)acrylate, and a combination thereof. Preferably, methyl (meth)acrylate or a combination of any one of the aforesaid monomers which monomer can give a homopolymer having a higher glass transition temperature(Tg), and another monomer of the aforesaid monomers which gives a homopolymer having a low Tg.

From the radically polymerizable silane compound of the formula (6), the aforesaid repeating unit (3) can be derived. Examples of the silane compound include γ-methacryloxypropyl -trimethoxysilane, γ-methacryloxypropyl -methyldimethoxysilane, γ-methacryloxypropyl-dimethylmethoxysilane, γ-methacryloxypropyl-triethoxysilane, γ-methacryloxypropyl -methyldiethoxysilane, γ-methacryloxypropyl-tributhoxysilane, γ-methacryloxypropyl-isopropenoxysilane, γ-acryloxypropyl-trimethoxysilane, γ-acryloxymethyl -trimethoxysilane, γ-acryloxypropyl-triethoxysilane, γ-acryloxypropylmethyl-diethoxysilane, styryltrimethoxysilane, styryltriethoxysilane, α-methylstyryltrimethoxysilane, and a mixture thereof. Preferably, γ-methacryloxypropyl -triethoxysilane is used.

A ratio of the monomers of the formulas (4), (5) and (6) is such that the organopolysiloxane compound of the formula (4) having a radically polymerizable group is in an amount of from 1 to 97 wt%, preferably from 5 to 90 wt%, the acrylic monomer of the formula (5) is in an amount of from 0 to 95 wt%, preferably from 2 to 60 wt%, and the radically polymerizable silane compound of the formula (6) is in an amount of from 1 to 10 wt%, preferably from 2 to 7 wt%, based on a total weight of the monomers. If the organopolysiloxane compound of the formula (4) is used less than the aforesaid lower limit, satisfactory effect by hair treating may not be obtained. More amount than the aforesaid upper limit of the organopolysiloxane compound may give a copolymer which does not adsorb on or react with the hair satisfactorily. If the acrylic monomer of the formula (5) is used more than the aforesaid upper limit, a copolymer having enough resistance to water may not be obtained. A less amount of the radically polymerizable silane compound of the formula (6) than the aforesaid lower limit may not give a copolymer having satisfactory reactivity with the hair. More amount than the aforesaid upper limit of the silane may give a copolymer which react among themselves in addition to with the hair. Taking the aforesaid matters into consideration, the ratio can be determined.

In addition to the aforesaid monomers, other radically polymerizable compound in an amount not to adversely affect the present invention can be copolymerized. Examples of the other radically copolymerizable monomer include carboxylic acids such as (meth) acrylic acid, fumaric acid and maleic acid; hydroxyalkyl esters such as hydroxyethyl (meth)acrylate and hydroxypropyl (meth)acrylate; acidic amides such as (meth)acrylamide; fluorinated alkyl esters such as perfluorooctylethyl (meth)acrylate, perfluorobutylethyl (meth)acrylate, styrene, acrylonitrile, vinyl acetate, vinylpyrrolidone, polyoxyethylene mono(meth)acrylate, polyoxypropylene mono(meth)acrylate, polyoxy(ethylene/propylene) mono(meth)acrylate, polycaprolactone mono(meth)acrylate, tris(trimethylsiloxy)silylpropyl methacrylate, and tris(trimethylsiloxy)silyl styrene.

Any method of copolymerisation can be used, for example, solution polymerization, emulsion polymerization, suspension polymerization, or mass polymerization, among which solution polymerization is preferred because of uniformity of a resulting polymer and easiness in molecular weight control. As a solvent for the solution polymerization, the one in which the aforesaid monomers and a polymer obtained can be uniformly dissolved. Examples of the solvent include toluene, xylene, ethanol, isopropanol, n-butanol, acetone, methyl ethyl ketone, methyl isobutyl ketone, ethyl acetate, n-butyl acetate, isobutyl acetate, diethyl ether, and tetrahydrofuran.

As a polymerization initiator, any conventionally used radical initiator may be used. Examples of the initiator include organic peroxides such as benzoylperoxide and dicumyl peroxide; and azo compounds such as azobisisobutylonitrile. A mercaptan derivative as a chain transfer agent may be used to control molecular weight of a polymer, for example, dodecyl mercaptan and mercaptopropyl trimethoxysilane. Use of a compound having a hydrolyzable group as aforementioned mercaptopropyl trimethoxysilane has an advantage that it adds a hydrolyzable silyl group to a polymer terminal. A solution of a resulting polymer can be used as such or may be diluted. Alternatively, the polymer is isolated or re-dissolved in another solvent for use as a hair treatment agent.

Preferably, the acryl/silicone copolymer obtained has a molecular weight, determined by GPC, of from 5,000 to 200,000, more preferably from 10,000 to 100,000. An acrylic-silicone copolymer having a lower molecular weight than the aforesaid lower limit may not show satisfactory effect in treating the hair. An acrylic-silicone copolymer having a higher molecular weight than the aforesaid upper limit may not give a hair cosmetic having a good usability.

In the present invention, the organopolysiloxane hair treatment agent (A) can be used in various ways. It can be used alone in the from of a dispersion or a solution in an organic solvent which is applied directly on the hair; it can be used in a two-agent kit composed of an aqueous or non-aqueous first agent selected from the group consisting of amino-modified silicone, amino acid-modified silicone and carboxyl-modified silicone, and the present hair treatment agent (A) as a second agent; and it can be used in a three-agent kit composed of a first agent comprising an aqueous or non-aqueous amino-modified silicone, a second agent comprising the present hair treatment agent (A), and a third agent comprising an aqueous or non-aqueous amino-modified silicone.

Preferably, an amino-modified silicone is used as the first agent and the present hair treatment agent is used as the non-aqueous second agent. Alternatively, an amino-modified silicone is used as the first agent, the present hair treatment agent is used as the non-aqueous second agent, and an aqueous or non-aqueous amino-modified silicone is used as the third agent. In both the two-agent and the three-agent kits, it was found that conditioning effect is maintained well by a synergistic effect of the amino-modified silicone and the present hair treatment agent.

Examples of the amino-modified silicone include those having amino groups grafted to a silicone backbone, those having an amino group bonded to either one end of a silicone backbone, those having amino groups bonded to both ends of a silicone backbone, those having amino groups at both ends of a silicone backbone and an amino group grafted to the silicone backbone, and those having a silicone side chain and an amino group both grafted to a silicone backbone. Preferably, the amino-modified silicone represented by the following formula is used.

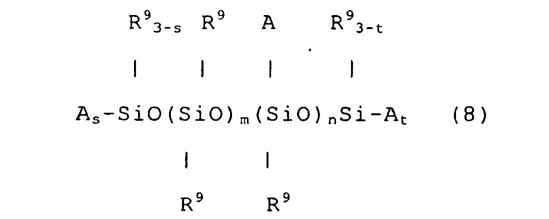

In the formula (8), A is -R¹⁰NH₂ or -R¹⁰NHR¹¹NH₂, wherein R¹⁰ and R¹¹ are alkylene groups having 1 to 8 carbon atoms,
R⁹ may be the same with or different from each other and is selected from the group consisting of alkyl groups having 1 to 30 carbon atoms, alicyclic groups, aryl groups, aralkyl groups, and fluorinated alkyl groups,
m and n may be the same with and different from each other and are integers of from 0 to 300, and s and t may be the same with or different from each other and are integers of from 0 to 3 with 1 ≤ n+s+t.

Examples of R⁹ include alkyl group such as methyl, ethyl, propyl, butyl, pentyl, hexyl, heptyl, octyl, nonyl, and decyl groups; alicyclic groups such as cyclopentyl and cyclohexyl groups; aryl groups such as phenyl and tolyl groups; aralkyl groups such as benzyl and phenetyl groups; and fluorinated alkyl groups such as trifluoropropyl and heptadecafluorodecyl groups, among which methyl, phenyl, and trifluoropropyl groups are preferred.

Examples of R¹⁰ and R¹¹ include methylene, ethylene, propylene, butylene, and pentylene groups, among which methylene, ethylene and propylene groups are preferred.

The present hair cosmetic may comprise various components contained in conventional cosmetics. Those components may be contained not only in one agent system composed of the component (A), but also in two- or three-agent kit as a whole, that is, the components may be incorporated in one or more of the agents from first to third one. These components will be explained below.

The present cosmetic may further comprise one or more of unctuous agent (B) . Any conventional unctuous agents can be used whether they are in the form of solid, semisolid or liquid. Examples of the unctuous agents include natural animal or plant oils, semi-synthetic oils, hydrocarbon oils, higher alcohol oils, ester oils and conventional silicone oils.

Examples of the natural animal or plant oils and semi-synthetic oils include avocado oil, linseed oil, almond oil, Chinese wax, perilla oil, olive oil, cacao butter, kapok wax, kaya oil, carnauba wax, liver oil, candellila wax, beef tallow, beef foot oil, beef bone fat, hydrogenated beef tallow, apricot kernel oil, spermaceti, hydrogenated oil, wheat germ oil, sesame oil, rice germ oil, rice branoil, sugarcane wax, sasanquaoil, safflower oil, shea butter, Chinese tung oil, cinnamon oil, jojoba wax, shellac wax, turtle oil, soybean oil, tea seed oil, tsubaki oil, evening primrose oil, corn oil, lard, rape seed oil, Japanese tung oil, rice-bran wax, germ oil, horse fat, persic oil, palm oil, palm kernel oil, castor oil, hydrogenated castor oil, caster oil fatty acid methyl ester, sunflower oil, grape seed oil, bayberry wax, jojoba oil, macadamia nut oil, beeds wax, mink oil, cottonseed oil, cotton wax, Japan wax, haze kernel oil, montan wax, coconut oil, hydrogenated coconut oil, tricoconut oil fatty acid glyceride, mutton-tallow, peanut oil, lanolin, liquid lanolin, reduced lanolin, lanolin alcohol, hard lanolin, lanolin acetate, lanolin fatty acid isopropyl, hexyl laurate, POE lanolin alcohol ether, POE lanolin alcohol acetate, lanolin fatty acid polyethylene glycol, POE hydrogenated lanolin alcohol ether, and egg yolk oil, wherein the term "POE" stands for polyoxyethylene.

Examples of the hydrocarbon oil include ozokerite, squalane, squalene, ceresine, paraffin, paraffin wax, liquid paraffin, pristane, polyisobutylene, microcrystalline wax and Vaseline; and those of a higher fatty acid which can be mixed include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid and 12-hydroxystearic acid.

Examples of the higher alcohol include lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, 2-decyltetradecinol, cholesterol, phytosterol, POE cholesterol ether, monostearyl glycerin ether (batyl alcohol) and monooleyl glyceryl ether (cerakyl alcohol).

Examples of the ester oil include diisobutyl adipate, 2-hexyldecyl adipate, di-2-heptylundecyl adipate, N-alkylglycol monoisostearates, isocetyl isostearate, trimethylolpropane triisostearic acid ester, ethylene glycol di-2-ethylhexanoic acid ester, cetyl 2-ethylhexanoate, trimethylolpropane tri-2-ethylhexanoic acid ester, pentaerythritol tetra-2-ethylhexanoic acid ester, cetyl octanoate, octyldodecyl gum ester, oleyl oleate, octyldodecyl oleate, decyl oleate, neopentyl glycol dicapric acid ester, triethyl citrate, 2-ethylhexyl cinnamate, amyl acetate, ethyl acetate, butyl acetate, isocetyl stearate, butyl stearate, diisopropyl sebacate, di-2-ethylhexyl sebacate, cetyl lactate, myristyl lactate, isopropyl palmitate, 2-ethylhexyl palmitate, 2-hexyldecyl palmitate, 2-heptylundecyl palmitate, cholesteryl 12-hydroxystearate, dipentaerythritol fatty acid esters, isopropyl myristate, octyldodecyl myristate, 2-hexyldecyl myristate, myristyl myristate, hexyldecyl dimethylocanoate, ethyl laurate, hexyl laurate, N-lauroyl-L-glutaminic acid 2-octyldodecyl ester and diisostearyl malic acid; and examples of glyceride oil which can be mixed therein include acetoglyceride, triisooctanoic acid glycride, triisostearic acid glyceride, triisopalmitic acid glyceride, tri-2-ethylhexanoic acid glyceride, monostearic acid glyceride, di-2-heptylundecanoic acid glyceride, trimyristic acid glyceride and myristic acid isostearic acid diglyceride.

Examples of the conventional silicone oil include organopolysiloxanes having a viscosity of from a low value to a high value, preferably from 0.65 to 1,000,000mm²/s, such as dimethylpolysiloxane, methylphenylpolysiloxane, methylhydrogenpolysiloxane and a copolymer of dimethylsiloxane and methylphenylsiloxane; cyclic siloxanes, such as octamethylcyclotetrasiloxane, decamethylcyclopentasiloxane, dodecamethylcyclohexasiloxane, tetramethyl-tetrahydrogencyclotetrasiloxane; silicone rubbers, such as gummy dimethylpolysiloxanes having high polymerization degrees and gummy dimethylsiloxane-methylphenylsiloxane copolymers having high polymerization degrees; and cyclosiloxane solutions of silicone rubber, trimethylsiloxysilicate, cyclosiloxane solutions of trimethylsiloxysilicate, higher alkoxy-modified silicones such as stearoxysilicone, higher fatty acid-modified silicones, alkyl-modified silicones, siliconols, fluorine-modified silicones, and solutions of silicone resins in a cyclic siloxane.

Examples of fluorine-containing oil include perfluoropolyether, perfluorodecalin and perfluorooctane.

A content of these unctuous agents (B) may vary depending on the form of the cosmetic and range from 0.01 to 99 wt% based on a total weight of the cosmetic.

The present hair cosmetic may further comprise (C) water according to a purpose of the cosmetic. A content of water (C) may vary depending on the form of the cosmetic and ranges from 0.01 to 99 wt% based on a total weight of the cosmetic.

The present cosmetic may further comprise one or more of a compound (D) having at least one alcoholic hydroxyl group per molecule, depending on a purposes of the cosmetic. Examples of the compoud (D) include lower alcohols, such as ethanol and isopropanol; sugar alcohols, such as sorbitol and maltose; sterols, such as cholesterol, sitosterol, phytosterol and lanosterol; and polyhydric alcohols such as butylene glycols, propylene glycols, dibutylene glycols, and pentylene glycols. The compound (D) may be incorporated in the cosmetic in an amount of from 0.1 to 98 wt% based on a total weight of the cosmetic.

The present cosmetic may further comprise one or more of a water-soluble or water-swellig polymer (E). Examples of the water-soluble or water-swellig polymer include plant origin polymers, such as gum arabic, tragacanth, galactan, carob gum, guar gum, karaya gum, carrageenan, pectin, agar, quince seed, starch (rice, corn, potato, wheat), alge colloid, tranto gum and locust bean gum; microbial polymers, such as xanthan gum, dextran, succinoglucan and pullulan; animal polymers, such as collagen, casein, albumin and gelatin; starch polymers, such as carboxymethyl starch and methylhydroxypropyl starch; cellulose polymers, such as methyl cellulose, ethyl cellulose, methylhydroxypropyl cellulose, carboxymethyl cellulose, hydroxymethyl cellulose, hydroxypropyl cellulose, nitrocellulose, sodium cellulose sulfate, sodium carboxymethylcellulose, crystalline cellulose and powdery cellulose; alginic acid polymers, such as sodium alginate and propylene glycol ester of alginic acid; vinyl polymers, such as polyvinyl methyl ether and carboxyvinyl polymer; polyoxyethylene polymers; polyoxyethylene-polyoxypropylene copolymers; acrylic polymers, such as sodium polyacrylate, polyethylacrylate and polyacrylamide; other synthetic water-soluble polymers, such as polyethyleneimines and cationic polymers; and inorganic water-soluble polymers, such as bentonite, aluminum magnesium silicate, montmorillonite, beidellite, nontronite, saponite, hectorite and silicic acid anhydride. The water-soluble polymer encompasses film-forming agents, such as polyvinyl alcohol and polyvinyl pyrrolidine, are also included. It may be incorporated in the cosmetic in an amount of from 0.1 to 25 wt% based on a total weight of the cosmetic.

The present hair cosmetic may further comprise one or more of a surfactant (F) according to a purpose of the cosmetic. Examples of the surfactant include anionic, cationic, nonionic and amphoteric surfactants and are not limited to a particuar one. Any surfactant can be used so long as it is used in ordinary cosmetics.

Examples of the anionic surfactant include fatty acid soap, such as sodium stearate or triethanolamine palmitate; alkyl ether carboxylic acids and salts thereof; salts of amino acid-fatty acid condensates; alkanesulfonates; alkenesulfonates; sulfonated fatty acid esters; sulfonated fatty acid amides; sulfonates of formaldehyde condensate type; alkylsulfates; higher secondary alcohol sulfates; alkyl and aryl ether sulfates; fatty acid ether sulfates, fatty acid alkylolamide sulfates; ether sulfates, such as Turkeky red oil; alkyl phosphates; ether phosphates; alkyl aryl ether phosphates; amide phosphates; and active agents of N-acylamino acid type.

Examples of the cationic surfactant include amine salts, such as alkylamie salts, polyamines and aminoalcohol fatty acid derivatives, quaternary alkylammonium salts, quaternary arylammonium salts, pyridinium salts and imidazolium salts.

Examples of the nonionic surfactant include sorbitan fatty acid esters, glycerin fatty acid esters, polyglycerin fatty acid esters, propylene glycol fatty acid esters, polyethylene glycol fatty acid esters, sucrose fatty acid esters, polyoxyethylene alkyl ethers, polyoxypropylene alkyl ethers, polyoxyethylene alkyl phenylethers, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hydrogenated castor oil, polyoxyethylene phytostanol ehter, polyoxyethylene phytosterol ether, polyoxyethylene cholestanol ether, polyoxyethylene cholesteryl ether, linear or branched polyoxyalkylene-modified organopolysiloxanes, linear or branched organopolysiloxanes modified with both polyoxyalkylene and alkyl groups, linear or branched polyglycerin-modified organopolysiloxanes, alkanolamides, sugar ethers and sugar amides; and examples of a usable amphoteric surfactant include betaine, aminocarboxylates and imdazoline derivatives. The surfactant may be incorporated in the cosmetic preferably in an amount of from 0.1 to 20 wt%, particularly 0.2 to 10 wt%, based on a total weight of the cosmetic.

The present cosmetic may further comprise one or more of a crosslinked organopolysiloxane (G), depending on a purpose of the cosmetic. The crosslinked organopolysiloxane preferably has been swelled with a silicone having a low viscosity of from 0.65 to 10.0 mm²/sec (25°C) in an amount larger than a weight of the organopolysiloxane (G). Prefered crosslinked organopolysiloxane is a reaction product of an organopolysiloxane having at least two alkenyl groups per molecule and an organohydrogenpolysiloxane having a Si-H bond. The crosslinked organopolysiloxane may have at least one moiety selected from polyoxyalkylene, polygrycerol, alkyl, alkenyl, aryl and fluoroalkyl moieties. The crosslinked organopolysiloxanes may be incorporated in the cosmetic preferably in an amount of from 0.1 to 50 wt%, more preferably from 1 to 30 wt%, based on a total weight of the cosmetic.

The present cosmetic may further comprise one or more of a conventional silicone resin (H) . Preferred silicone resin is an acryl/silicone graft or block copolymer without having a hydrolyzable silyl group. Use may be made of an acryl/silicone resin having no hydrolysable silyl group and at least one moiety selected from the group consisting of pyrrolidone moieties, long-chain alkyl moieties, polyoxyalkylenemoieties, fluoroalkyl moieties and carboxylic acid moieties.

Preferably, this silicone resin is a silicone compound having a network structure represented as MQ, MDQ, MT, MDT, or MDTQ. Use may be made of a silicone network compound having at least one moiety selected from the group consisting of pyrrolidone, long-chain alkyl, polyoxyalkylene, fluoroalkyl, and amino moieties.

The silicone resin may be incorporated in the cosmetic preferably in an amount of from 0.1 to 20 wt%, more preferably from 1 to 10 wt%.

The present cosmetic may further comprise one or more of powder and/or coloring agent (I) depending on a purpose of the cosmetic. Any powder conventionally used in cosmetics can be used irrespective of its form, for example, spherical, acicular or tabular; its size, for example, fume state, fine grain or pigment grade; and its structure, for example, porous or nonporous. Examples include inorganic powder, organic powder, metal salt surfactant powder, colored pigments, pearl pigments, metallic powder pigments and natural pigments.

Examples of the inorganic powder include titanium dioxide, zirconiumoxide, zincoxide, ceriumoxide, magnesiumoxide, barium sulfate, calcium sulfate, magnesium sulfate, calcium carbonate, magnesium carbonate, talc, mica, kaolin, sericite, muscovite, synthetic mica, phlogopite, ruby mica, biotite, lipidolite, silicic acid, silicic acid anhydride, aluminum silicate, magnesium silicate, aluminum magnesium silicate, calcium silicate, barium silicate, strontium silicate, metal salts of tungstic acid, hydroxyapatite, vermiculite, haidilite, bentonite, montmorillonite, hectorite, zeolite, ceramics powder, calcium secondary phosphate, alumina, aluminum hydroxie, boron nitride and silica.

Examples of the organic powder include polyamide powder, polyester powder, polyethylene powder, polypropylene powder, polystyrene powder, polyurethane powder, benzoguanamine powder, polymethylbenzoguanamine powder, polytetrafluoroethylene powder, polymethylmethacrylate powder, cellulose powder, silk powder, nylon powder such as 12-nylon powder or 6-nylon powder, crosslinked dimethylsilicone fine powder, polymethylsilsesquioxane fine powder, powder of crosslinked silicone composite with polymethylsilsesquioxane bonded to the silicone, styrene-acrylic acid copolymer powder, divinylbenzene-styrene copolymer powder, vinyl resin powder, urea resin powder, phenol resin powder, fluororesin powder, silicone resin powder, acrylic resin powder, melamine resin powder, epoxy resin powder, polycarbonate resin powder, microcrystalline fiber powder, starch powder and lauroyl lysine powder.

Examples of the metal salt surfactant powder (metal soap powder) include powders of zinc stearate, aluminum stearate, calcium stearate, magnesium stearate, zinc myristate, magensium myristate, zinc cetylphosphate, calcium cetylphosphate and zinc sodium cetylphosphate.

Examples of the colored pigment include red pigments, such as iron oxide, iron hydroxide and iron titanate; inorganic brown pigments, such as γ-iron oxide; inorganic yellow pigments, such as iron oxide yellow and loess; inorganic black pigments, such as iron oxide black and carbon black; inorganic violet pigments, such as manganese violet and cobalt violet; inorganic green pigments, such as chromium hydroxide, chromium oxide, cobalt oxide and cobalt titanate; inorganic blue pigments, such as Prussian blue and ultramarine blue; lakes of tar pigments; lakes of natural dyes; and a composite powder of two or more of above.

Examples of the pearl pigment include titanium oxide-coatedmica, bismuth oxychloride, titanium oxide-coated bismuth oxychloride, titanium oxide-coated talc, fish scales, and titanium oxide-coated colored mica. Examples of the metallic powder pigment include aluminum powder, copper powder and stainless powder. Examples of the tar pigment include Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206 and Orange No. 207; and examples of the natural pigment include powders of carminic acid, laccaic acid, carthamin, bradilin and crocin.

The powder may be used as in a composite form or may be treated with a commonly used oil, silicone oil, fluorocompound, or surfactant. The powder may be used as a mixture of two or more thereof, if desired. The powder is incorporated in the cosmetic preferably in an amount of from 0.1 to 99 wt% based on a total weight of the cosmetic.

To the present cosmetic may further comprise ingredients used in general cosmetics such as oil-soluble gelling agents, clay minerals modified with organic compounds, resins, ultraviolet absorbents or scattering material, moisturizing agents, antiseptics, antimicrobial agents, perfume, salts, antioxidants, pH regulators, chelating agents, refrigerant, anti-inflammatory agents, skin beautifying components (a skin whitener, a cell activator, a rough dry skin improver, a blood circulation promoter, a skin astringent and an anti-seborrheic agent), vitamins, amino acids, nucleic acids, hormones and clathrate compounds,and polymers for hair setting , in an amount not to have adverse influences on the effects of the present invention.

Examples of the oil-soluble gelling agent include metal soaps, such as aluminum stearate, magnesium stearate and zinc myristate; amino acid derivatives, such as N-lauroyl-L-glutamic acid and α,γ-di-n-butylamine; dextrin fattyacidesters, such as dextrinpalmitic acid ester, dextrin stearic acid ester and dextrin 2-ethylhexaminic acid palmitic acid ester; sucrose fatty acid esters, such as sucrose palmitic acid ester and sucrose stearic acid ester; benzylidene derivatives of sorbitol, such as monobenzylidene sorbitol and dibenzylidene sorbitol; and clay minerals modified with organic compounds, such as dimethylbenzyldodecyl ammonium montmorillonite clay and dimethyldioctadecyl ammonium montmorillonite clay.

Examples of the ultraviolet light absorbent include those of benzoic acid type, such as p-aminobenzoic acid; those of anthranilic acid type, such as methyl anthranilate; those of salicylic acid type, such as methyl salicylate; those of succinic acid type, such as octyl p-methoxysuccinate; those of benzophenone type, such as 2,4-dihydroxybenzophenone; those of urocanic acid type, such as ethyl urocanate; and those of dibenzoylmethane type, such as 4-t-butyl-4'-methoxydibenzoylmethane. Examples of ultraviolet scattering material include titanium oxide fine powder, fine powder of titanium oxide containing iron, zinc oxide fine powder, cerium oxide fine powder, a composed powder of two or more of these and powder which absorbs and scatters ultraviolet.

Examples of the moisturizing agent include glycerin, sorbitol, propylene glycol, dipropylene glycol, 1,3-butylene glycol, glucose, xylitol, maltitol, polyethyleneglycol, hyaluromicacid, chondroitin sulfuric acid, pyrrolidone carboxylic acid, polyoxyethylene glycoside, and polyoxypropylene methylglycoside.

Examples of the antiseptic agent include alkyl p-hydroxybenzoates, benzoic acid, sodium benzoate, sorbic acid, potassium sorbate and phenoxyethanol; and those of an antimicrobial agent which can be added include benzoic acid, salicylic acid, carbolic acid, sorbic acid, alkyl p-hydroxybenzoates, p-chlorometacresol, hexachlorophene, benzalkonium chloride, chlorhexidine chloride, trichlorocarbanilide, photosensitizer and phenoxyethanol.

Examples of the antioxidant include tocopherol, butylhydroxyanisole, dibutylhydroxytoluene and phytic acid; those of a pH regulator which can be added include lactic acid, citricacid, glycolic acid, succinicacid, tartaricacid, dl-malic acid, potassium carbonate, sodium hydrogen carbonate and ammonium hydrogen carbonate; those of a chelating agent which can be added include alanine, sodium ethylenediaminetetraacetate, sodium polyphosphate, sodium metaphosphate and phosphoric acid; those of a refrigerant which can be added include L-menthol and camphor; and those of an anti-inflammatory agent which can added include allantoin, glycyrrhizin and salts thereof, glycyrrhetinic acid and stearyl glycyrrhetinate, tranexamic acid and azulene.

Examples of the skin-beautifying component include whitening agents, such as placenta extract, arbutin, glutathione and Yukinoshita extract; cell activators, such as royal jelly, photosensitizer, cholesterol derivatives and calf blood extract; rough dry skin improvers; blood circulation improvers, such as nonylic acid vanillyl amide, benzyl nicotinate, β-butoxyethyl nicotinate, capsaicin, zingerone, cantharis tincture, ichtammol, caffeine, tannicacid, α-borneol, tocopheryl nicotinate, inositol hexanicotinate, cyclandelate, cinnarizine, tolazoline, acetyl choline, verapamil, cepharanthin and γ-oryzanol; skin astringents, such as zinc oxide and tannic acid; and anti-seborrheic agents, such as sulfur and thianthol.

Examples of the vitamin include vitamin A, such as vitamin A oil, retinol, retinyl acetate and retinyl palmitate; vitamin B, including vitamin B2 such as riboflavin, riboflavin butyrate and flavin adenine nucleotide, vitamin B6 such as pyridoxine hydrochloride, pyridoxine dioctanoate and pyridoxine tripalmitate, vitamin B12 and its derivatives, and vitamin B15 and its derivatives; vitamin C, such as L-ascorbic acid, L-ascorbic acid dipalmitic ester, sodium (L-ascorbic acid) -2-sulfate and dipotassium L-ascorbic acid diphosphate; vitamin D, such as ergocalciferol and cholecarciferol; vitamin E, such as α-tocopherol, β-tocopherol, γ-tocopherol, dl-α-tocopheryl acetate, dl-α-tocopherylnicotinate and dl-α-tocopheryl succinate; vitamin H; vitamin P; nicotinic acids, such as nicotinic acid, benzyl nicotinate and nicotinic acid amide; pantothenic acids, such as calcium pantothenate, D-pantothenyl alcohol, pantothenyl ethyl ether and acetylpantothenyl ethyl ether; and biotin.

Examples of the amino acid include glycine, valine, leucine, isoleucine, serine, threonine, phenylaranine, alginine, lysine, aspartic acid, aspartic acid, glutamic acid, cystine, cysteine, methionine, and tryptophan; those of a nucleic acid which can be added include deoxyribonucleic acid; and those of hormone which can be added include estradiol and ethenyl estradiol.

Examples of the polymer for hair setting include amphoteric, anionic, cationic, and nonionic polymers, such as polymers of polyvinyl pyrrolidone type such as polyvinyl pyrrolidone, vinyl pyrrolidone /vinyl acetate copolymers; acidic polymers of vinyl acetate ether type such as methyl vinyl ether / maleic acid anhydride alkyl half ester copolymer; polymers of acidic poly vinyl acetate type such as vinyl acetate / crotonic acid copolymer; acidic acrylic polymers such as (meth) acrylic acid / alkyl (meth) acrylate copolymer, (meth)acrylic acid / alkyl (meth) acrylate / alkyl acrylic amide copolymer, and amphoteric acrylic polymer such as N-methacryloylethyl-N,N-dimethylammonium alpha-N-methylcarboxybetaine / alkylmetahcrylate copolymer, hydroxypropyl (metha)acrylate/butylaminoethyl methacrylate / octyl amide of acrylic acid copolymer. Use is also made of naturally occurring polymers such as cellulose or derivatives thereof, keratin, collagen and derivatives thereof.

The present hair cosmetic may be in the form of liquid, milky lotion, cream, solid, paste, gel, multilayer, mousse, spray or stick.

### EXAMPLES

The present invention will be explained in detail with reference to Examples, but not limited thereto. In the following, "%" means % by weight unless otherwise specified.

### Synthesis Example 1

In a glass flask provided with a stirrer, a thermometer and a reflux condenser, 50 wt% of the radically polymerizable organopolysiloxane of the following formula (9), 5 wt% of methyl methacrylate, 40 wt% of stearyl methacrylate, 5 wt% of γ-methacryloxypropyl-trimethoxysilane, toluene in the same weight as a total weight of the aforesaid monomers, and azobisisobutylonitrile in an amount of 1% by weight of a total weight of the monomers and toluene were placed and subjected to polymerization by heating under nitrogen stream at 100 °C for 10 hours. Then, toluene was distilled off at a reduced pressure. An acryl/silicone graft copolymer for the hair thus obtained was light yellow solid having a melting point of 30°C and a weight average molecular weight, determined by GPC, of 42,000 in polystyrene equivalent.

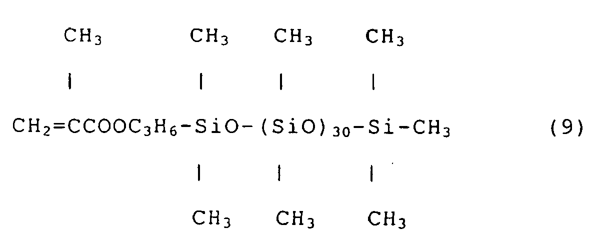

### Synthesis Example 2

An acryl/silicone graft copolymer was prepared as in Example 1 by copolymerizing 50 wt% of the radically polymerizable organopolysiloxane of the above formula (9), 30 wt% of methyl methacrylate, 7.5 wt% of butyl methacrylate, 7.5 wt% of 2-ethyl hexylacrylate and 5 wt% of γ-methacryloxypropyl-triethoxysilane. The copolymer obtained was transparent light yellow resin having a softening point of 93°C and a weight average molecular weight, determined by GPC, of 55,000 in polystyrene equivalent.

### Synthesis Example 3

An acryl/silicone graft copolymer was prepared as in Example 1 by copolymerizing 90 wt% of the radically polymerizable organopolysiloxane of the following formula (10), 50 wt% of methyl methacrylate, and 5 wt% of γ-methacryloxypropyl-triethoxysilane. The copolymer for the hair obtained was colorless transparent liquid having a viscosity at 25°C of 270 mm²/sec, a specific gravity of 0.981, a refractive index of 1.4152 and a weight average molecular weight, determined by GPC, of 11,000 in polystyrene equivalent.

### Example 1. Hair spray (one-agent type)

| Components | wt % |
|---|---|
| 1. Synthesis Example 2 | 5.0 |
| 2. Ethanol | 95.0 |

The components described above were mixed. The mixture was packed in a spray container to prepare a hair spray. The hair spray obtained was tested by five female panelists and rated according to the following criteria by comparing the hair to which the hair spray was applied with the reference hair which was not applied with the hair spray in terms of easiness to comb, moisturized feel, softness, and gloss.

| Mark | Easiness to comb | Moisturized feel, softness, and gloss |
|---|---|---|
| 5 | Much easier | Significant |
| 4 | A little easier | A little more than the reference level |
| 3 | Reference level | Reference level |
| 2 | A little difficult than the reference | A little less than the reference level |
| 1 | Much difficult | None |

The marks were averaged and used for evaluation according to the criteria shown below.

### Evaluation according to an averaged mark:

| Averaged mark | |
|---|---|
| 4.5 or higher | A |
| 3.5 to below 4.5 | B |
| 2.5 to below 3.5 | C |
| 1.5 to below 2.5 | D |
| below 1.5 | E |

### Comparative Example 1

A hair spray was prepared as in Example 1 except that a methylhydrogenpolysiloxane was used in place of the organopolysiloxane prepared in Synthetic Example 2.

Results of Example 1 and Comparative Example 1 are as shown in Table 1 below.

**Table 1**

| | Example 1 | Comparative Example 1 |
|---|---|---|
| Easiness to comb | A | A |
| Moisturized feel | A | B |
| Softness | A | A |
| Gloss | A | B |

As is evident from Table 1, the hair treated with the hair spray of the present invention was easy to comb, moisturized, soft and glossy compared with the hair treated with methylhydrogenpolysiloxane.

### Examples 2-5, Comparative Examples 2-4

Hair treatment agents each having the formulation shown in Table 2 were prepared.

**Table 2**

| First agent | | Ex^{*3}.2 | Ex.3 | Ex.4 | Ex.5 | Comp.Ex.^{*4} 2 | Comp. Ex.3 | Comp. Ex.4 |
|---|---|---|---|---|---|---|---|---|
| 1 | Amino-modified silicone^{*1} | 5 | 5 | 5 | - | 5 | 5 | 5 |
| 2 | Cetanol | 8 | 8 | 8 | - | 8 | 8 | 8 |
| 3 | Stearyl trimethyl ammonium chloride | 3.5 | 3.5 | 3.5 | - | 3.5 | 3.5 | 3.5 |
| 4 | Glycerin | 5 | 5 | 5 | - | 5 | 5 | 5 |
| 5 | Purified water | 78.5 | 78.5 | 78.5 | - | 78.5 | 78.5 | 78.5 |

| Second agent | | | | | | | | |
|---|---|---|---|---|---|---|---|---|
| 1 | Synthesis example 1 | 6 | - | - | 6 | - | - | - |
| 2 | Synthesis example 3 | - | 6 | 6 | - | - | - | - |
| 3 | Synthesis example 4 | - | - | 6 | - | - | - | - |
| 4 | Methylhydrogen polysiloxane | - | - | - | - | 6 | 6 | - |
| 5 | Ethanol | 94 | 94 | 94 | 94 | 94 | 94 | 100 |

| Third agent | | | | | | | | |
|---|---|---|---|---|---|---|---|---|
| 1 | Amino-modified silicone^{*1} | - | - | 2 | - | - | 2 | - |
| 2 | Highly polymerized methylpoly -siloxane solution | - | - | 5 | - | - | 5 | - |
| 3 | Cetanol | - | - | 8 | - | - | 8 | - |
| 4 | Stearyl trimethyl ammonium chloride | - | - | 3.5 | - | - | 3.5 | - |
| 5 | Glycerin | - | - | 3 | - | | 3 | - |
| 6 | Purified water | - | - | - 78.5 | - | - | 78.5 | 100 |

| | | | | | | | | |
|---|---|---|---|---|---|---|---|---|
| ^{*}1 Amino-modified silicone :KF-8005, ex Shin-Etsu Chemical Co.,Ltd. *2 Solution of a Highly polymerized methylpolysiloxane: KF-9013, ex Shin-Etsu Chemical Co.,Ltd. *3 "Ex." stands for Example. *4 "Comp.Ex." stands for Comparative Example. | | | | | | | | |

Each hair treatment agent was evaluated using hair bundles for testing.

The hair bundle for testing was a bundle of ordinary hair with a length of 20 cm and a total weight of 6 . 0 g which had been shampooed and breached with a breaching agent.

In Examples 2 and 3, the hair bundle was coated with one gram of a first agent followed by washing with water, and then coated with one gram of a second agent followed by drying; in Example 4, the hair bundle was treated with a first agent and then a second agent as described above, and then coated with one gram of a third agent; and in Example 5, the hair bundle was washed and then coated with one gram of a second agent. Each of thus treated hair bundle was processed under the conventional conditions by treating with a conventional hair treatment product, rinsing and drying.

Subsequently, the process consisting of shampooing, hair treatment with a treatment product and drying was repeated 20 times consecutively.

The hair bundles immediately after treated with the present hair treatment agent and those after 20 cycles of the processing were compared with as ordinary hair being a reference in easiness to comb, moisturized feel, softness, and gloss. Results are as shown in the following Table.

**Table 3**

| Immediately after treatment | Example 2 | Example 3 | Example 4 | Example 5 | Comp.Ex.2 | Comp.Ex.3 | Comp.Ex.4 |
|---|---|---|---|---|---|---|---|
| Easiness to comb | A | A | A | A | B | A | B |
| Moisturized feel | A | A | A | A | B | B | B |
| Softeness | A | A | A | A | A | A | B |
| Gloss | A | A | A | A | B | A | B |

| After 20-cycle processing | | | | | | | |
|---|---|---|---|---|---|---|---|
| Easiness to comb | A | A | A | B | D | C | D |
| Moisturized feel | A | A | A | A | D | B | D |
| Softeness | A | A | A | A | D | B | D |
| Gloss | A | A | A | B | D | C | D |

As shown in Table 3, compared with the hair cosmetic of Comparative Examples, the hair treatment cosmetics of the present invention were superior in all the aspects, i.e., easiness to comb, moisturizing effect, softening effect and gloss, indicating improvement in durability of hair treatment applied to the hair.

In Examples 2 to 4, the hair after 20-cycle processing showed the same result as that of the hair immediately after treated, indicating excellent resistance to shampooing. Especially, Examples 2 and 5, wherein the present treatment agent is used together with the amino-modified silicone, durable coating film on the hair was formed to maintain smoothness in combing and gloss, compared with Comparative Example 4. Although, the effect was demonstrated by using amino-modified silicones, the same effect is attained with amino acid-modified silicones and carboxyl-modified silicones hair treatment agent.

## Claims

1. A hair treatment agent comprising an acryl/silicone copolymer (A) having at least one hydrolyzable silyl group per molecule.

2. The hair treatment agent according to claim 1, wherein the acryl/silicone copolymer (A) is an acryl/silicone graft copolymer having an acrylic polymer backbone and an organopolysiloxane polymer branch.

3. The hair treatment agent according to claim 1 or 2, wherein the hydrolyzable silyl group is
-Si(CH₃)₃₋ₘ(OR)ₘ
wherein R is an alkyl group having 1 to 10 carbon atoms, an alicyclic group, an alkenyl group, an aryl group, an aralkyl group, or an alkaryl group, m is an integer of from 1 to 3, and R may be different from each other when m is 2 or 3.

4. The hair treatment agent according to any one of claims 1 to 3, wherein the acryl/silicone copolymer (A) has the following repeating units (1), (2) and (3): 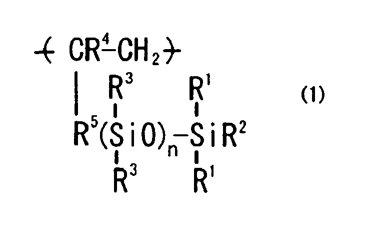 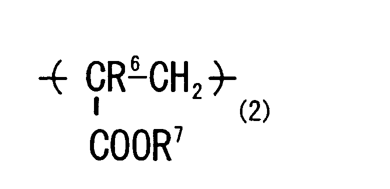 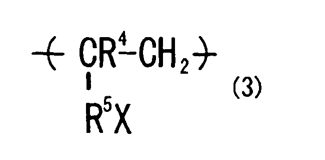 wherein R¹, R² and R³ may be the same with or different from each other and are selected from the group consisting of alkyl groups having 1 to 30 carbon atoms, aryl groups, aralkyl groups and fluorinated alkyl groups,
R⁴ and R⁶ may be the same with or different from each other and are selected from the group consisting of a hydrogen atom and a methyl group,
R⁵ may be the same with or different from each other and is an alkyleneoxycarbonyl group having 2 to 11 carbon atoms or a phenylene group,
R⁷ is an alkyl group having 1 to 30 carbon atoms,
X is the hydrolyzable silyl group as described in claim 1 or claim 3, and
n is an integer of from 3 to 500.

5. The hair treatment agent according to any one of claims 1 to 4, wherein the copolymer has a weight average molecular weight of from 5,000 to 200,000.

6. A hair cosmetic comprising the hair treatment agent according to any one of claims 1 to 5.

7. A two-agent hair cosmetic kit composed of a first agent comprising at least one selected from the group consisting of amino-modified silicones, amino acid-modified silicones, and carboxyl-modified silicones, and a second agent comprising the hair treatment agent according to any one of claims 1 to 5.

8. A three-agent hair cosmetic kit composed of the two agents hair cosmetic kit according to claim 7 and an amino-modified silicone.

9. The cosmetic according to claim 7 or 8, wherein the amino-modified silicone is at least one selected from the group consisting of silicones having an amino group grafted to a silicone backbone, silicones having an amino group bonded to either one end of a silicone backbone, silicones having amino groups bonded to both ends of a silicone backbone, silicones having amino groups bonded to both ends of a silicone backbone and an amino group grafted to the silicone backbone, and silicones having a silicone chain and an amino group both grafted to a silicone backbone.

10. The cosmetic according to any one of claims 7 to 9, wherein the amino-modified silicone is represented by the following formula (8), 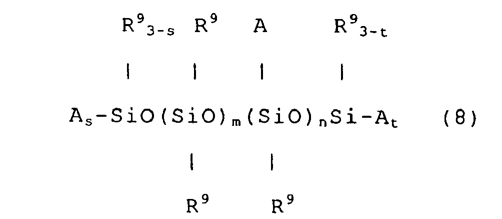 wherein A is -R¹⁰NH₂ or -R¹⁰NHR¹¹NH₂, wherein R¹⁰ and R¹¹ are alkylene groups having 1 to 8 carbon atoms,
R⁹ may be the same with or different from each other and is selected from the group consisting of alkyl groups having 1 to 30 carbon atoms, alicyclic groups, aryl groups, aralkyl groups, and fluorinated alkyl groups,
m and n may be the same with and different from each other and are integers of from 0 to 300, and s and t may be the same with or different from each other and are integers of from 0 to 3 with 1 ≤ n+s+t.

11. The hair cosmetic according to any one of claims 6 to 10, wherein the hair cosmetic further comprises one or more of an unctuous agent (B).

12. The hair cosmetic according to claim 11, wherein at least part of the unctuous agent (B) is selected from the group consisting of organopolysiloxanes having a viscosity of from 0.65 to 1,000,000 mm²/s, cyclic siloxanes, and silicone rubbers or resins dissolved or dispersed in a cyclic siloxane.

13. The hair cosmetic according to claim 11 or 12, wherein at least part the aforesaid unctuous agent (B) has a fluorine-containing group.

14. The hair cosmetic according to any one of claims 6 to 13, wherein the hair cosmetic further comprises water (C).

15. The hair cosmetic according to any one of claims 6 to 14, wherein the hair cosmetic further comprises a compound (D) having an alcoholic hydroxyl group in a molecule.

16. The hair cosmetic according to any one of claims 6 to 15, wherein the hair cosmetic further comprises a water-soluble or water-swelling polymer (E).

17. The hair cosmetic according to any one of claims 6 to 16, wherein the hair cosmetic further comprises a surfactant (F).

18. The hair cosmetic according to claim 17, wherein the surfactant (F) is a linear or branched polyoxyalkylene-modified organopolysiloxane.

19. The hair cosmetic according to claim 17, wherein the surfactant (F) is a linear or branched polyglycerin-modified organopolysiloxane.

20. The hair cosmetic according to any one of claims 6 to 19, wherein the hair cosmetic further comprises a crosslinked organopolysiloxane (G).

21. The hair cosmetic according to claim 20, wherein the crosslinked organopolysiloxane (G) has been incorporated in the cosmetic in a state swollen in a silicone having a viscosity of from 0.65 to 10.0 mm²/sec in an amount larger than a weight of the crosslinked organopolysiloxane (G).

22. The hair cosmetic according to claim 20 or 21, wherein the crosslinked organopolysiloxane (G) is a reaction product of an organopolysiloxane having at least two alkenyl groups per molecule with an organohydrogenpolysiloxane having a Si-H bond.

23. The hair cosmetic according to any one of claims 20 to 22, wherein the crosslinked organopolysiloxane (G) has at least one moiety selected from the group consisting of polyoxyalkylene moieties, polyglycerin moieties, alkyl moieties, alkenyl moieties, aryl moieties and fluoroalkyl moieties.

24. The hair cosmetic according to any one of claims 6 to 23, wherein the cosmetic further comprises a silicone resin (H) selected from the group consisting of acrylsilicone resins which do not have a hydrolyzable silyl group, silicone network compounds represented as MQ, MDQ, MT, MDT, or MDTQ, and silicone network compounds having at least one moiety selected from the group consisting of pyrrolidone moieties, long-chain alkyl moieties, polyoxyalkylene moieties, fluoroalkyl moieties, and amino moieties.

25. The hair cosmetic according to any one of claims 6 to 24, wherein the cosmetic further comprises one or more of powder and/or coloring agent (I).

26. The hair cosmetic according to claim 25, wherein at least part of the powder and/or coloring agent (I) is crosslinked silicone fine powder with a structure of crosslinked dimethylsilicone, polymethylsilsesquioxane fine powder, silica made hydrophobic, or composite fine powder composed of spherical silicone rubber coated with polymethylsilsesquioxane particles.

27. The hair cosmetic according to any one of claims 6 to 26, wherein the cosmetic is in the form of liquid, milky lotion, cream, solid, paste, gel, multilayer, mousse, spray or stick.

28. A method to prepare the hair treatment agent according to any one of claims 1 to 5, wherein the method comprising the step of copolymerizing 1 to 97 wt % of an organopolysiloxane represented by the following formula (4) having a radically polymerizable group, 0 to 95 wt % of an acrylicmonomer represented by the following formula (5), and 1 to 10 wt% of a silane compound represented by the following formula (6) having a radically polymerizable group, 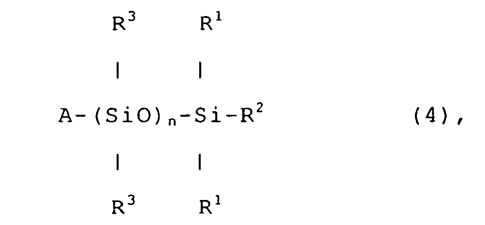 wherein R¹, R² and R³ may be the same with or different from each other and are selected from the group consisting of alkyl groups having 1 to 30 carbon atoms, aryl groups, aralkyl groups and fluorinated alkyl groups, A is a radically polymerizable group represented by the following formula (7),
CH₂=C(R⁴)R⁵- (7),
wherein R⁴ is a hydrogen atom or a methyl group and R⁵ is an alkyleneoxycarbonyl group having 2 to 11 carbon atoms or a phenylene group, and
n is an integer of from 3 to 500;
CH₂=C (R⁶) COOR⁷ (5),
wherein R⁶ is a hydrogen atom or a methyl group and R⁷ is an alkyl group having 1 to 30 carbon atoms;
B-Si (CH₃) ₃₋ₘ (OR⁸) ₘ (6),
wherein B is selected from the groups defined for A above, independently of A, R⁸ is an alkyl group having 1 to 4 carbon atoms or an alkenyl group, m is an integer of from 1 to 3, and R⁸'s may be different from each other when m is 2 or 3.

## Patentansprüche

1. Haarbehandlungsmittel, das ein Acryl/Silikon-Copolymer (A), das wenigstens eine hydrolisierbare Silylgruppe pro Molekül hat, umfasst.

2. Haarbehandlungsmittel gemäß Anspruch 1, wobei das Acryl/Silikon-Copolymer (A), ein Acryl/Silikon-Pfropfcopolymer ist, das eine Acrylpolymer-Hauptkette und eine Organopolysiloxanpolymer-Verzweigung hat.

3. Haarbehandlungsmittel gemäß Anspruch 1 oder 2, wobei die hydrolisierbare Silylgruppe
-Si(CH₃)₃₋ₘ(OR)ₘ
ist, worin R eine Alkylgruppe mit 1 bis 10 Kohlenstoffatomen, eine alicyclische Gruppe, eine Alkenylgruppe, eine Arylgruppe, eine Aralkylgruppe oder eine Alkarylgruppe ist, m eine ganze Zahl von 1 bis 3 ist und R voneinander verschieden sein können, wenn m 2 oder 3 ist.

4. Haarbehandlungsmittel gemäß einem der Ansprüche 1 bis 3, wobei das Acryl/Silikon-Copolymer (A) die folgenden Repetiereinheiten (1), (2) und (3) hat: 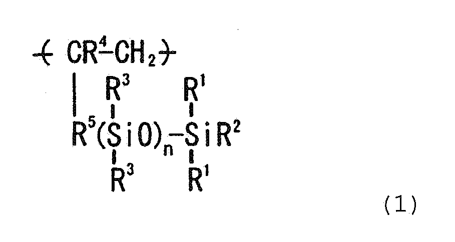 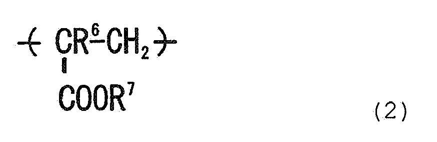 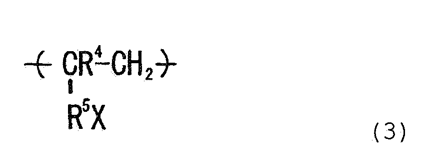 worin R¹, R² und R³ gleich oder voneinander verschieden sein können und ausgewählt sind aus der Gruppe, bestehend aus Alkylgruppen mit 1 bis 30 Kohlenstoffatomen, Arylgruppen, Aralkylgruppen und fluorierten Alkylgruppen,
R⁴ und R⁶ gleich oder voneinander verschieden sein können und ausgewählt sind aus der Gruppe, bestehend aus einem Wasserstoffatom und einer Methylgruppe,
R⁵ gleich oder voneinander verschieden sein können und eine Alkylenoxycarbonylgruppe mit 2 bis 11 Kohlenstoffatomen oder eine Phenylengruppe ist,
R⁷ eine Alkylgruppe mit 1 bis 30 Kohlenstoffatomen ist,
X die hydrolisierbare Silylgruppe, wie in Anspruch 1 oder Anspruch 3 beschrieben, ist und
n eine ganze Zahl von 3 bis 500 ist.

5. Haarbehandlungsmittel gemäß einem der Ansprüche 1 bis 4, wobei das Copolymer ein gewichtsmittleres Molekulargewicht von 5.000 bis 200.000 hat.

6. Haarkosmetikum, das das Haarbehandlungsmittel gemäß einem der Ansprüche 1 bis 5 umfasst.

7. Zwei-Komponenten-Haarkosmetikum-Kit, bestehend aus einem ersten Mittel, das wenigstens eines, ausgewählt aus der Gruppe, bestehend aus Amino-modifizierten Silikonen, Aminosäuremodifizierten Silikonen und Carboxyl-modifizierten Silikonen, umfasst, und einem zweiten Mittel, das das Haarbehandlungsmittel gemäß einem der Ansprüche 1 bis 5 umfasst.

8. Drei-Komponenten-Haarkosmetikum-Kit, bestehend aus dem Zwei-Komponenten-Haarkosmetikum-Kit gemäß Anspruch 7 und einem Amino-modifizierten Silikon.

9. Kosmetikum gemäß Anspruch 7 oder 8, wobei das Amino-modifizierte Silikon wenigstens eines ist, ausgewählt aus der Gruppe, bestehend aus Silikonen, die eine Aminogruppe auf eine Silikonhauptkette gepfropft haben, Silikonen, die eine Aminogruppe an ein Ende einer Silikonhauptkette gebunden haben, Silikonen, die Aminogruppen an beiden Enden einer Silikonhauptkette gebunden haben, Silikonen, die Aminogruppen an beiden Enden einer Silikonhauptkette gebunden und eine Aminogruppe auf die Silikonhauptkette gepfropft haben, und Silikonen, die eine Silikonkette und eine Aminogruppe beide auf die Silikonhauptkette gepfropft haben.

10. Kosmetikum gemäß einem der Ansprüche 7 bis 9, wobei das Amino-modifizierte Silikon durch die folgende Formel (8) dargestellt wird 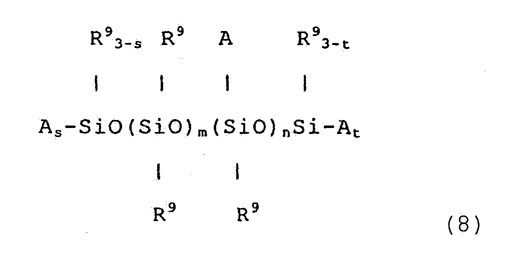 worin A -R¹⁰NH₂ oder -R¹⁰NHR¹¹NH₂ ist, worin R¹⁰ und R¹¹ Alkylengruppen mit 1 bis 8 Kohlenstoffatomen sind,
R⁹ gleich oder voneinander verschieden sein können und ausgewählt ist aus der Gruppe, bestehend aus Alkylgruppen mit 1 bis 30 Kohlenstoffatomen, alicyclischen Gruppen, Arylgruppen, Aralkylgruppen und fluorierten Alkylgruppen,
m und n gleich oder voneinander verschieden sein können und ganze Zahlen von 0 bis 300 sind und s und t gleich oder voneinander verschieden sein können und ganze Zahlen von 0 bis 3 sind, wobei 1 ≤ n + s + t.

11. Haarkosmetikum gemäß einem der Ansprüche 6 bis 10, wobei das Haarkosmetikum außerdem ein öliges Mittel oder mehrere ölige Mittel (B) umfasst.

12. Haarkosmetikum gemäß Anspruch 11, wobei wenigstens ein Teil des öligen Mittels (B) ausgewählt ist aus der Gruppe, bestehend aus Organopolysiloxanen mit einer Viskosität von 0,65 bis 1.000.000 mm²/s, cyclischen Siloxanen und Silikonkautschuken oder -harzen, gelöst oder dispergiert in einem cyclischen Siloxan.

13. Haarkosmetikum gemäß Anspruch 11 oder 12, wobei wenigstens ein Teil des öligen Mittels (B) eine fluorhaltige Gruppe hat.

14. Haarkosmetikum gemäß einem der Ansprüche 6 bis 13, wobei das Haarkosmetikum außerdem Wasser (C) umfasst.

15. Haarkosmetikum gemäß einem der Ansprüche 6 bis 14, wobei das Haarkosmetikum außerdem eine Verbindung (D), die eine alkoholische Hydroxylgruppe in einem Molekül hat, umfasst.

16. Haarkosmetikum gemäß einem der Ansprüche 6 bis 15, wobei das Haarkosmetikum außerdem ein wasserlösliches oder in Wasser quellendes Polymer (E) umfasst.

17. Haarkosmetikum gemäß einem der Ansprüche 6 bis 16, wobei das Haarkosmetikum außerdem ein oberflächenaktives Mittel (F) umfasst.

18. Haarkosmetikum gemäß Anspruch 17, wobei das oberflächenaktive Mittel (F) ein lineares oder verzweigtes Polyoxyalkylen-modifiziertes Organopolysiloxan ist.

19. Haarkosmetikum gemäß Anspruch 17, wobei das oberflächenaktive Mittel (F) ein lineares oder verzweigtes Polyglycerinmodifiziertes Organopolysiloxan ist.

20. Haarkosmetikum gemäß einem der Ansprüche 6 bis 19, wobei das Haarkosmetikum außerdem ein vernetztes Organopolysiloxan (G) umfasst.

21. Haarkosmetikum gemäß Anspruch 20, wobei das vernetzte Organopolysiloxan (G) in einem Zustand, gequollen in einem Silikon, das eine Viskosität von 0.65 bis 10,0 mm²/s hat, in einer Menge, die größer ist als das Gewicht des vernetzten Organopolysiloxans (G), in das Kosmetikum eingearbeitet wurde.

22. Haarkosmetikum gemäß Anspruch 20 oder 21, wobei das vernetzte Organopolysiloxan (G) ein Reaktionsprodukt eines Organopolysiloxans, das wenigstens zwei Alkenylgruppen pro Molekül hat, mit einem Organohydrogenpolysiloxan, das eine Si-H-Bindung hat, ist.

23. Haarkosmetikum gemäß einem der Ansprüche 20 bis 22, wobei das vernetzte Organopolysiloxan (G) wenigstens eine Gruppierung, ausgewählt aus der Gruppe, bestehend aus Polyoxyalkylengruppierungen, Polyglyceringruppierungen, Alkylgruppierungen, Alkenylgruppierungen, Arylgruppierungen und Fluoralkylgruppierungen, hat.

24. Haarkosmetikum gemäß einem der Ansprüche 6 bis 23, wobei das Kosmetikum außerdem ein Silikonharz (H), ausgewählt aus der Gruppe, bestehend aus Acrylsilikonharzen, die keine hydrolisierbare Silylgruppe haben, Silikonnetzwerkverbindungen, dargestellt als MQ, MDQ, MT, MDT oder MDTQ, und Silikonnetzwerkverbindungen, die wenigstens eine Gruppierung haben, ausgewählt aus der Gruppe, bestehend aus Pyrrolidongruppierungen, langkettigen Alkylgruppierungen, Polyoxyalkylengruppierungen, Fluoralkylgruppierungen und Aminogruppierungen, umfasst.

25. Haarkosmetikum gemäß einem der Ansprüche 6 bis 24, wobei das Kosmetikum außerdem eines oder mehrere aus Pulver und/oder Färbemittel (I) umfasst.

26. Haarkosmetikum gemäß Anspruch 25, wobei wenigstens ein Teil des Pulvers und/oder des Färbemittels (I) feines Pulver aus vernetztem Silikon mit einer Struktur von vernetztem Dimethylsilikon, feines Polymethylsilsesquioxan-Pulver, hydrophob gemachtes Siliciumdioxid oder feines Verbundmaterialpulver, bestehend aus sphärischem Silikonkautschuk, überzogen mit Polymethylsilsesquioxanpartikeln, ist.

27. Haarkosmetikum gemäß einem der Ansprüche 6 bis 26, wobei das Kosmetikum in der Form einer Flüssigkeit, einer milchigen Lotion, einer Creme, eines Feststoffs, einer Paste, eines Gels, einer Multilayer, eines Schaums, eines Sprays oder eines Stifts ist.

28. Verfahren zur Herstellung des Haarbehandlungsmittels gemäß einem der Ansprüche 1 bis 5, wobei das Verfahren den Schritt eines Copolymerisierens von 1 bis 97 Gew.-% eines Organopolysiloxans, dargestellt durch die folgende Formel (4), das eine radikalisch polymerisierbare Gruppe hat, 0 bis 95 Gew.-% eines Acrylmonomers, dargestellt durch die folgende Formel (5), und 1 bis 10 Gew.-% einer Silanverbindung, dargestellt durch die folgende Formel (6), die eine radikalisch polymerisierbare Gruppe hat, 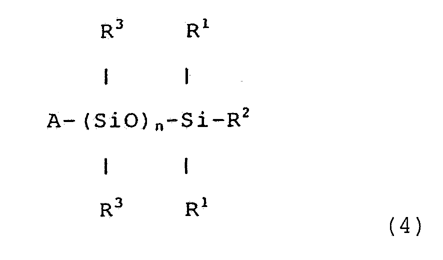 worin R¹, R² und R³ gleich oder voneinander verschieden sein können und ausgewählt sind aus der Gruppe, bestehend aus Alkylgruppen mit 1 bis 30 Kohlenstoffatomen, Arylgruppen, Aralkylgruppen und fluorierten Alkylgruppen, A eine radikalisch polymerisierbare Gruppe, dargestellt durch die folgende Formel (7), ist
CH₂=C(R⁴)R⁵- (7)
worin R⁴ ein Wasserstoffatom oder eine Methylgruppe ist und R⁵ eine Alkylenoxycarbonylgruppe mit 2 bis 11 Kohlenstoffatomen oder eine Phenylengruppe ist und
n eine ganze Zahl von 3 bis 500 ist;
CH₂=C (R⁶) COOR⁷ (5)
worin R⁶ ein Wasserstoffatom oder eine Methylgruppe ist und R⁷ eine Alkylgruppe mit 1 bis 30 Kohlenstoffatomen ist;
B-Si(CH₃)₃₋ₘ(OR⁸)ₘ. (6)
worin B ausgewählt ist aus den oben für A definierten Gruppen, unabhängig von A, R⁸ eine Alkylgruppe mit 1 bis 4 Kohlenstoffatomen oder eine Alkenylgruppe ist, m eine ganze Zahl von 1 bis 3 ist und R⁸ voneinander verschieden sein können, wenn m 2 oder 3 ist.

## Revendications

1. Agent de traitement capillaire comprenant un copolymère acryle/silicone (A) ayant au moins un groupe silyle hydrolysable par molécule.

2. Agent de traitement capillaire selon la revendication 1, dans lequel le copolymère acryle/silicone (A) est un copolymère greffé acryle/silicone ayant un squelette polymère acrylique et une ramification polymère organopolysiloxane.

3. Agent de traitement capillaire selon la revendication 1 ou 2, dans lequel le groupe silyle hydrolysable est
-Si(CH₃)₃₋ₘ(OR)ₘ
où R est un groupe alkyle ayant 1 à 10 atomes de carbone, un groupe alicyclique, un groupe alcényle, un groupe aryle, un groupe aralkyle ou un groupe alkaryle, m est un entier valant 1 à 3, et R peut être différent des autres lorsque ion vaut 2 ou 3.

4. Agent de traitement capillaire selon l'une quelconque des revendications 1 à 3, dans lequel le copolymère acryle/silicone (A) a les motifs répétés (1), (2) et (3) suivants : 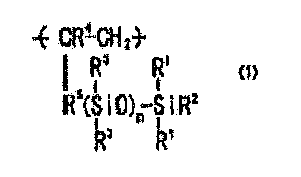 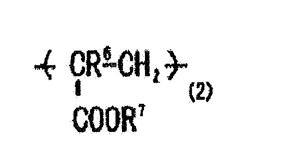 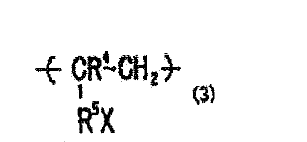 où R¹, R² et R³ peuvent être identiques ou différents les uns des autres et sont choisis dans le groupe consistant en les groupes alkyle ayant 1 à 30 atomes de carbone, les groupes aryle, les groupes aralkyle et les groupes alkyle fluorés,
R⁴ et R⁶ peuvent être identiques ou différents les uns des autres et sont choisis dans le groupe consistant en un atome d'hydrogène et un groupe méthyle,
R⁵ peut être identique ou différent des autres et est un groupe alkylèneoxycarbonyle ayant 2 à 11 atomes de carbone ou un groupe phénylène,
R⁷ est un groupe alkyle ayant 1 à 30 atomes de carbone,
X est le groupe silyle hydrolysable comme décrit dans la revendication 1 ou la revendication 3, et
n est un entier valant 3 à 500.

5. Agent de traitement capillaire selon l'une quelconque des revendications 1 à 4, dans lequel le copolymère a une masse moléculaire moyenne en poids de 5 000 à 200 000.

6. Cosmétique capillaire comprenant l'agent de traitement capillaire selon l'une quelconque des revendications 1 à 5.

7. Nécessaire cosmétique capillaire à deux agents composé d'un premier agent comprenant au moins un élément choisi dans le groupe consistant en les silicones modifiées par un groupe amino, les silicones modifiées par un acide aminé et les silicones modifiées par un groupe carboxyle, et d'un second agent comprenant l'agent de traitement capillaire selon l'une quelconque des revendications 1 à 5.

8. Nécessaire cosmétique capillaire à trois agents composé du nécessaire cosmétique capillaire à deux agents selon la revendication 7 et d'une silicone modifiée par un groupe amino.

9. Cosmétique selon la revendication 7 ou 8, dans lequel la silicone modifiée par un groupe amino est au moins un élément choisi dans le groupe consistant en les silicones ayant un groupe amino greffé à un squelette silicone, les silicones ayant un groupe amino lié à l'une des extrémités d'un squelette silicone, les silicones ayant des groupes amino liés aux deux extrémités d'un squelette silicone, les silicones ayant des groupes amino liés aux deux extrémités d'un squelette silicone et un groupe amino greffé au squelette silicone, et les silicones ayant une chaîne silicone et un groupe amino, tous deux greffés à un squelette silicone,

10. Cosmétique selon l'une quelconque des revendications 7 à 9, dans lequel la silicone modifiée par un groupe amino est représentée par la formule (8) suivante, 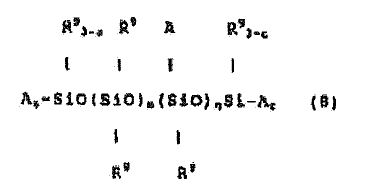 dans laquelle A est -R¹⁰NH₂ ou -R¹⁰NHR¹¹NH₂, où R¹⁰ et R¹¹ sont des groupes alkylène ayant 1 à 8 atomes de carbone,
R⁹ peut être identique ou différent des autres et est choisi dans le groupe consistant en les groupes alkyle ayant 1 à 30 atomes de carbone, les groupes alicyclique, les groupes aryle, les groupe aralkyle et les groupes alkyle fluorés,
m et n peuvent être identiques et différents l'un de l'autre et sont des entiers valant 0 à 300, et s et t peuvent être identiques et différents l'un de l'autre et sont des entiers valant 0 à 3 avec 1 ≤n+s+t.

11. Cosmétique capillaire selon l'une quelconque des revendications 6 à 10, dans lequel le cosmétique capillaire comprend en outre un ou plusieurs agents onctueux (B).

12. Cosmétique capillaire selon la revendication 11, dans lequel au moins une partie de l'agent onctueux (B) est choisie dans le groupe consistant en les organopolysiloxanes ayant une viscosité de 0,65 à 1 000 000 mm²/s, les siloxanes cycliques et les caoutchoucs ou résines de silicone dissous ou dispersés dans un siloxane cyclique.

13. Cosmétique capillaire selon la revendication 11 ou 12, dans lequel au moins une partie de l'agent onctueux (B) précité a un groupe contenant du fluor.

14. Cosmétique capillaire selon l'une quelconque des revendications 6 à 13, dans lequel le cosmétique capillaire comprend en outre de l'eau (C).

15. Cosmétique capillaire selon l'une quelconque des revendications 6 à 14, dans lequel le cosmétique capillaire comprend en outre un composé (D) ayant un groupe hydroxyle alcoolique dans une molécule.

16. Cosmétique capillaire selon l'une quelconque des revendications 6 à 15, dans lequel le cosmétique capillaire comprend en outre un polymère soluble dans l'eau ou gonflant dans l'eau (E).

17. Cosmétique capillaire selon l'une quelconque des revendications 6 à 36, dans lequel le cosmétique capillaire comprend en outre un agent tensioactif (F).

18. Cosmétique capillaire selon la revendication 17, dans lequel l'agent tensioactif (F) est un organopolysiloxane modifié par polyoxyalkylène linéaire ou ramifié.

19. Cosmétique capillaire selon la revendications 17, dans lequel l'agent tensioactif (F) est un organopolysiloxane modifié par polyglycérine linéaire ou ramifié.

20. Cosmétique capillaire selon l'une quelconque des revendications 6 à 19, dans lequel le cosmétique capillaire comprend en outre un organopolysiloxane réticulé (G).

21. Cosmétique capillaire selon la revendication 20, dans lequel l'organopolysiloxane réticulé (G) a été incorporé dans le cosmétique dans un état gonflé dans une silicone ayant une viscosité de 0,65 à 10,0 mm²/s en une quantité supérieure à un poids de l'organopolysiloxane réticulé (G).

22. Cosmétique capillaire selon la revendication 20 ou 21, dans lequel l'organopolysiloxane réticulé (G) est un produit de réaction d'un organopolysiloxane ayant au moins deux groupes alcényle par molécule avec un organohydrogénopolysiloxane ayant une liaison Si-H.

23. Cosmétique capillaire selon l'une quelconque des revendications 20 à 22, dans lequel l'organopolysiloxane réticulé (G) a au moins une fraction choisie dans le groupe consistant en les fractions polyoxyalkylène, les fractions polyglycérine, les fractions alkyle, les fractions alcényle, les fractions aryle et les fractions fluoroalkyle.

24. Cosmétique capillaire selon l'une quelconque des revendications 6 à 23, dans lequel le cosmétique comprend en outre une résine de silicone (H) choisie dans le groupe consistant en les résines d'acrylsilicone qui n'ont pas de groupe silyle hydrolysable, les composés de réseau de silicone représentés en tant que MQ, MDQ, MT, MDT ou MDTQ, et les composés de réseau de silicone ayant au moins une fraction choisie dans le groupe consistant en les fractions pyrrolidone, les fractions alkyle à chaîne longue, les fractions polyoxyalkylène, les fractions fluoroalkyle et les fractions amino.

25. Cosmétique capillaire selon l'une quelconque des revendications 6 à 24, dans lequel le cosmétique comprend en outre un ou plusieurs éléments parmi une poudre et/ou un agent colorant (I).

26. Cosmétique capillaire selon la revendication 25, dans lequel au moins une partie de la poudre et/ou de l'agent colorant (I) est une fine poudre de silicone réticulée avec une structure de diméthylsilicone réticulée, une fine poudre de polyméthylsilsesquioxane, de la silice rendue hydrophobe, ou une fine poudre composite composée d'un caoutchouc de silicone sphérique revêtu de particules de polyméthylsilsesquioxane.

27. Cosmétique capillaire selon l'une quelconque des revendications 6 à 26, dans lequel le cosmétique est sous la forme d'un liquide, d'une lotion lactée, d'une crème, d'un solide, d'une pâte, d'un gel, d'une multicouche, d'une mousse, d'une pulvérisation ou d'un bâton.

28. Procédé de préparation de l'agent de traitement capillaire selon l'une quelconque des revendications 1 à 5, dans lequel le procédé comprend l'étape de copolymérisation de 1 à 97 % en poids d'un organopolysiloxane représenté par la formule (4) suivante ayant un groupe radicalement polymérisable, de 0 à 95% en poids d'un monomère acrylique représenté par la formule (5) suivante, et de 1 à 10 % en poids d'un composé silane représenté par la formule (6) suivante ayant un groupe radicalement polymérisable, 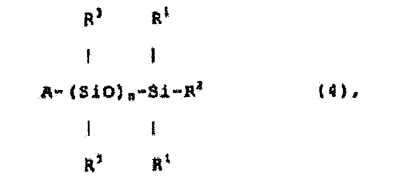 dans laquelle R¹, R² et R³ peuvent être identiques ou différents les uns des autres et sont choisis dans le groupe consistant en les groupes alkyle ayant 1 à 30 atomes de carbone, les groupes aryle, les groupes aralkyle et les groupes alkyle fluorés, A est un groupe radicalement polymérisable représenté par la formule (7) suivante,
CH₂=C(R⁴)R⁵ - (7),
dans laquelle R⁴ est un atome d'hydrogène ou un groupe méthyle et R⁵ est un groupe alkylèneoxycarbonyle ayant 2 à 11 atomes de carbone ou un groupe phénylène, et n est un entier valant 3 à 500 ;
CH₂=C(R⁶)COOR⁷ (5),
dans laquelle R⁶ est un atome d'hydrogène ou un groupe méthyle et R⁷ est un groupe alkyle ayant 1 à 30 atomes de carbone ;
B-Si(CH₃)₃₋ₘ(OR⁸)ₘ (6),
dans laquelle B est choisi dans les groupes définis pour A ci-dessus, indépendamment de A, R⁸ est un groupe alkyle ayant 1 à 4 atomes de carbone ou un groupe alcényle, m est un entier valant 1 à 3, et les R⁸ peuvent être différents les uns des autres lorsque m vaut 2 ou 3.
